# EUROPÄISCHE PATENTSCHRIFT

(11) **EP 3 062 719 B1**
(45) Veröffentlichungstag und Bekanntmachung des Hinweises auf die Patenterteilung: **18.04.2018**
(21) Anmeldenummer: 14784230.6
(22) Anmeldetag: 14.10.2014
(51) Int. Cl.: A61B 18/00, A61B 17/00

(54) **ELEKTROCHIRURGISCHER ROHRSCHAFT, CHIRURGISCHER INSTRUMENTENGRIFF UND ELEKTROCHIRURGISCHES ROHRSCHAFTINSTRUMENT**
ELECTROSURGICAL TUBULAR SHAFT, SURGICAL HANDLE AND ELECTROSURGICAL TUBULAR SHAFT INSTRUMENT
ARBRE TUBULAIRE ÉLECTROCHIRURGICAL, POIGNÉE D'INSTRUMENT CHIRURGICAL ET INSTRUMENT ÉLECTROCHIRURGICAL À ARBRE TUBULAIRE

(30) Priorität: 29.10.2013 DE 102013111912
(43) Veröffentlichungstag der Anmeldung: 07.09.2016
(73) Patentinhaber: Aesculap AG, 78532 Tuttlingen (DE)
(72) Erfinder: WAND, Matthias, 78532 Tuttlingen (DE); WEISSHAUPT, Dieter, 78194 Immendingen (DE); NESPER, Markus, 78532 Tuttlingen (DE)
(74) Vertreter: Hoeger, Stellrecht & Partner Patentanwälte mbB
(86) Internationale Anmeldenummer: PCT/EP2014/072044
(87) Internationale Veröffentlichungsnummer: WO 2015/062862

(56) Entgegenhaltungen:
- DE-A1-102006 007 828
- DE-A1-102006 052 407
- DE-A1-102011 056 003
- US-A- 6 004 320

## Beschreibung

Die Erfindung betrifft einen elektrochirurgischen gemäß Anspruch 1 für die Verwendung mit einem chirurgischen Instrumentengriff zur Ausbildung eines elektrochirurgischen Rohrschaftinstrumentes, wobei der Rohrschaft ein Rohr umfasst, ein distalseitig am Rohrschaft angeordnetes elektrochirurgisches Werkzeug, mindestens eine mit dem Werkzeug in elektrischer Verbindung stehende und im Rohr verlaufende elektrische Anschlussleitung sowie mindestens ein proximalseitig am Rohrschaft angeordnetes Kopplungselement zum Zusammenwirken mit mindestens einem korrespondierenden Kopplungselement des Instrumentengriffes zu dessen lösbarer Kopplung mit dem Rohrschaft, wobei der Rohrschaft ein proximalseitig an der mindestens einen Anschlussleitung angeordnetes elektrisches Anschlusselement für eine elektrische Energieversorgung umfasst.

Außerdem betrifft die Erfindung einen chirurgischen Instrumentengriff gemäß Anspruch 11 für die Verwendung mit einem elektrochirurgischen Rohrschaft zur Ausbildung eines elektrochirurgischen Rohrschaftinstrumentes, umfassend mindestens ein Kopplungselement zum Zusammenwirken mit mindestens einem korrespondierenden Kopplungselement des Rohrschaftes zu dessen lösbarer Kopplung mit dem Instrumentengriff.

Ferner betrifft die Erfindung gemäß Anspruch 12 ein elektrochirurgisches Rohrschaftinstrument, umfassend einen chirurgischen Instrumentengriff sowie einen elektrochirurgischen Rohrschaft, der lösbar mit dem Instrumentengriff verbindbar ist. "Proximal" und "distal" sind vorliegend auf einen bestimmungsgemäßen Gebrauch des Rohrschafts, des Instrumentengriffes und des Rohrschaftinstrumentes bezogen aufzufassen, wobei der Rohrschaft proximalseitig und der Instrumentengriff distalseitig zusammenwirken. Der Benutzer wirkt von proximal auf den Instrumentengriff ein und operiert mit dem distal am Rohrschaft angeordneten Werkzeug.

Ein Rohrschaft und ein Instrumentengriff der vorstehend genannten Art können über die eine Kopplungseinrichtung ausbildenden jeweiligen Kopplungselemente miteinander lösbar verbunden werden, um ein vorstehend genanntes elektrochirurgisches Rohrschaftinstrument zu bilden. Üblicherweise umfasst der Instrumentengriff zu diesem Zweck eine hülsenförmige Aufnahme, in die der Rohrschaft teilweise einführbar und in der er lösbar verrastbar ist. Mittels mindestens eines Griffelementes des Instrumentengriffes kann üblicherweise auf ein im Rohr geführtes Zug- und/oder Schubkraftübertragungselement eingewirkt werden, das distalseitig mit dem Werkzeug gekoppelt ist, so dass dieses mit dem mindestens einen Griffelement betätigt werden kann.

Das Werkzeug des Rohrschafts ist mit mindestens einer elektrischen Anschlussleitung elektrisch verbunden, so dass dem Werkzeug elektrochirurgische Energie zugeführt werden kann. Aus Gründen der elektrischen Sicherheit verläuft die mindestens eine Anschlussleitung im Rohr, wodurch sie auch vom Operationsfeld ferngehalten werden kann, was die Handhabbarkeit des Rohrschaftes verbessert. Bei den bekannten Rohrschaftinstrumenten wird die mindestens eine elektrische Anschlussleitung ebenso wie das mindestens eine Kopplungselement in den Instrumentengriff eingeführt, in dem sie durch elektrische Kontaktglieder kontaktiert ist. Zu diesem Zweck ist der Instrumentengriff seinerseits über eine weitere elektrische Anschlussleitung mit einer elektrischen Energieversorgung verbunden. Diese Rohrschäfte, Instrumentengriffe und damit Rohrschaftinstrumente weisen einen verhältnismäßig komplizierten Aufbau auf, da zur mechanischen Verbindung des Rohrschafts mit dem Instrumentengriff und zur elektrischen Kontaktierung der mindestens einen Anschlussleitung im Instrumentengriff nur verhältnismäßig geringer Bauraum zur Verfügung steht. Bei Wechselrohrschaftsystemen, bei denen ein wiederverwendbarer Instrumentengriff mit Einwegrohrschäften zum Einsatz kommt, erschwert eine derartige mechanische Kopplung und elektrische Kontaktierung im Instrumentengriff dessen Reinigung, insbesondere Sterilisation, nach dem operativen Einsatz.

Die DE 10 2011 056 003 A1, auf welcher der Oberbegriff von Anspruch 11 beruht, beschreibt einen chirurgischen Instrumentengriff. Ein gattungsgemäßer Rohrschaft gemäß des Oberbegriffs von Anspruch 1 ist in der DE 10 2006 052 407 A1 offenbart.

Aufgabe der vorliegenden Erfindung ist es, einen gattungsgemäßen Rohrschaft, einen gattungsgemäßen Instrumentengriff und ein gattungsgemäßes Rohrschaftinstrument bereitzustellen, so dass das aus Rohrschaft und Instrumentengriff gebildete Rohrschaftinstrument einen konstruktiv einfacheren Aufbau aufweist.

Diese Aufgabe wird bei einem Rohrschaft der eingangs genannten Art erfindungsgemäß dadurch gelöst, dass das Rohr eine distal des mindestens einen Kopplungselementes angeordnete Austrittsöffnung umfasst, durch die hindurch die mindestens eine Anschlussleitung aus dem Rohr austritt.

Bei dem erfindungsgemäßen Rohrschaft verläuft die mindestens eine elektrische Anschlussleitung vom Werkzeug in distal-proximaler Richtung im Rohr. Durch die Austrittsöffnung hindurch kann die mindestens eine Anschlussleitung in distalem Abstand zu dem mindestens einen Kopplungselement aus dem Rohr austreten. Die mindestens eine Anschlussleitung weist somit einen im Rohr verlaufenden distalen Abschnitt auf sowie einen außerhalb des Rohres verlaufenden proximalen Abschnitt. Proximalseitig ist an der mindestens einen Anschlussleitung ein elektrisches Anschlusselement angeordnet, das mit einer elektrischen Energieversorgung verbindbar ist, so dass dem Werkzeug über die mindestens eine Anschlussleitung elektrochirurgische Energie zugeführt werden kann. Durch den Verlauf des distalen Abschnittes der mindestens einen Anschlussleitung im Rohr kann die elektrische Sicherheit des Rohrschaftes gewährleistet werden, und die Anschlussleitung ist fern vom Operationsfeld angeordnet, um die Handhabung des Rohrschaftes zu erleichtern. Die Anordnung des proximalen Abschnittes der mindestens einen Anschlussleitung außerhalb des Rohres erleichtert deren elektrische Kontaktierung erheblich. Die Anschlussleitung kann außen am Instrumentengriff vorbei oder diesen entlang geführt und außerhalb des Instrumentengriffes kontaktiert werden. Insbesondere braucht ein mit dem erfindungsgemäßen Rohrschaft zusammenwirkender Instrumentengriff keine innenliegenden elektrischen Kontaktglieder zu umfassen. Das Anschlusselement des Rohrschaftes kann infolge der Anordnung außerhalb des Rohres und des Instrumentengriffes hinreichend groß ausgestaltet werden, so dass es von einem Benutzer auf einfache Weise handhabbar ist. Eine filigrane Ausgestaltung elektrischer Kontaktglieder wie bei gattungsgemäßen Rohrschäften und Instrumentengriffen kann dadurch vermieden werden. Dies erlaubt es auch, dem Rohrschaft und dem Instrumentengriff eine robustere Konstruktion zu verleihen.

Die Austrittsöffnung ist günstigerweise in der Nähe des proximalen Endes des Rohres angeordnet, so dass die mindestens eine Anschlussleitung über eine möglichst große Strecke im Rohr verläuft. Beispielsweise ist die Austrittsöffnung bei in den Instrumentengriff eingreifendem Rohr im Wesentlichen unmittelbar distal des Instrumentengriffes angeordnet.

Bevorzugt tritt die mindestens eine Anschlussleitung seitlich aus dem Rohr aus, quer zur einer vom Rohr definierten Richtung. Dies gibt die Möglichkeit, dass das Rohr mit einem proximal der Austrittsöffnung angeordneten Abschnitt in den Instrumentengriff eingreifen kann, beispielsweise zur Ankopplung an diesen.

Bei einer konstruktiv einfachen Ausgestaltung des Rohrschaftes ist die Austrittsöffnung eine im Rohr gebildete Durchbrechung, durch die hindurch die mindestens eine Anschlussleitung aus dem Rohr austritt.

Von Vorteil ist es, wenn der Rohrschaft eine das Rohr aufnehmende Buchse zum Einführen in eine korrespondierende Hülse des Instrumentengriffes umfasst, welche Buchse proximalseitig zumindest ein Kopplungselement umfasst, und wenn in der Buchse eine Durchbrechung gebildet ist, durch die die mindestens eine Anschlussleitung hindurchgeführt ist. Die Buchse ermöglicht über das mindestens eine Kopplungselement das Ankoppeln des Rohrschaftes am Instrumentengriff, dessen Hülse zu diesem Zweck ein korrespondierendes Kopplungselement aufweisen kann. Über die Buchse kann das Rohr versteift und dadurch geschützt werden. Die aus dem Rohr ausgetretene mindestens eine Anschlussleitung ist durch die in der Buchse gebildete Durchbrechung hindurchgeführt, die günstigerweise mit der Austrittsöffnung am Rohr fluchtet.

Vorzugsweise umfasst der Rohrschaft eine die mindestens eine Anschlussleitung an deren aus dem Rohr ausgetretenen Abschnitt zumindest abschnittsweise umgebende Umhüllung. Die Umhüllung ermöglicht einen Schutz und/oder eine Führung der mindestens einen Anschlussleitung. Beispielsweise ist die Umhüllung als Kanal oder als Tülle ausgestaltet, in dem bzw. der die mindestens eine Anschlussleitung geführt ist. Die Umhüllung kann die Anschlussleitung formschlüssig umgeben und an dieser fixiert sein. Es kann vorgesehen sein, dass die mindestens eine Anschlussleitung im Bereich der Austrittsöffnung nicht von der Umhüllung umgeben ist, so dass die mindestens eine Anschlussleitung zur vereinfachten Handhabung ihres aus dem Rohr ausgetretenen Abschnittes geknickt oder gebogen werden kann. Proximalseitig der Austrittsöffnung ist die Anschlussleitung demgegenüber bevorzugt von der Umhüllung umgeben.

Die Umhüllung ist bevorzugt längserstreckt ausgebildet, insbesondere geradlinig.

Vorteilhafterweise ist die Umhüllung biegesteif, so dass die mindestens eine Anschlussleitung auf bessere Weise geschützt werden kann. Unter "biegesteif" wird vorliegend insbesondere verstanden, dass die zum Biegen der Umhüllung erforderliche Kraft die zum Biegen der mindestens einen Anschlussleitung erforderliche Kraft wesentlich übersteigt.

Bei einer andersartigen vorteilhaften Ausführungsform ist die Umhüllung flexibel, zum Beispiel ist sie ein flexibler Schlauch.

Eine bevorzugte Ausführungsform des erfindungsgemäßen Rohrschafts ist ein bipolarer elektrochirurgischer Rohrschaft und umfasst zwei mit dem Werkzeug in elektrischer Verbindung stehende elektrische Anschlussleitungen. Das Werkzeug umfasst beispielsweise zwei Maulteile, von denen jedes mit einer der Anschlussleitungen elektrisch verbunden ist. Die Maulteile können dadurch mit unterschiedlicher elektrischer Polarität versehen werden. Durch Energiezufuhr zum Rohrschaft kann zwischen den Maulteilen ein elektrischer Strom fließen, insbesondere ein Hochfrequenzstrom, um Körpergewebe zu koagulieren.

Das Werkzeug kann, beispielsweise bei der zuletzt erwähnten bevorzugten Ausführungsform des erfindungsgemäßen Rohrschafts, ein Pinzettenwerkzeug sein, wobei die beiden Maulteile mit Anschlussleitungen unterschiedlicher Polarität verbunden sind.

Ist mehr als eine Anschlussleitung vorhanden, ist es von Vorteil, wenn ein gemeinsames Anschlusselement vorgesehen ist, das proximalseitig an beiden Anschlussleitungen angeordnet ist. Dies erlaubt es, beide Anschlussleitungen proximalseitig durch ein gemeinsames Anschlusselement mit einer Energieversorgung zu verbinden.

Weiter kann vorgesehen sein, dass die zwei (oder mehr) elektrischen Anschlussleitungen an ihren aus dem Rohr ausgetretenen Abschnitten von einer gemeinsamen Umhüllung umgeben sind. Zwei (oder mehr) Anschlussleitungen können zum Schutz und/oder zur vereinfachten Handhabung außerhalb des Rohres durch die Umhüllung zusammengefasst werden.

Es kann vorgesehen sein, dass der Rohrschaft mindestens ein im Anschlusselement gefasstes, mit der mindestens einen Anschlussleitung elektrisch verbundenes Kontaktglied aufweist. Beispielsweise ist das Anschlusselement als Stecker ausgebildet, der einen das mindestens eine Kontaktglied einfassenden Körper aufweist. Beispielsweise ist das mindestens eine Kontaktglied in den Körper eingegossen und darin mit der mindestens einen Anschlussleitung elektrisch verbunden. Bei Vorhandensein von zwei oder mehr elektrischen Anschlussleitungen sind günstigerweise zwei oder mehr Kontaktglieder im Anschlusselement gefasst und mit jeweils einer der Anschlussleitungen elektrisch verbunden.

Das mindestens eine elektrische Kontaktglied kann an einer proximalen Stirnseite des Anschlusselementes angeordnet sein, und es kann sich axial oder achsparallel zu einer vom Rohrschaft definierten proximal-distalen Richtung erstrecken. Bei dem mindestens einen Kontaktglied kann es sich um ein männliches oder weibliches Kontaktglied handeln.

Die mindestens eine Anschlussleitung kann lösbar mit dem Anschlusselement, in dem das mindestens eine Kontaktglied gefasst ist, verbindbar sein. Beispielsweise kann die mindestens eine Anschlussleitung über eine elektrische Steckverbindung mit dem Anschlusselement verbunden werden und dadurch das mindestens eine Kontaktglied elektrisch kontaktieren.

Von Vorteil ist es, wenn der Rohrschaft mindestens ein Verbindungselement umfasst zum lösbaren Festlegen der mindestens einen Anschlussleitung am Instrumentengriff. Der aus dem Rohr ausgetretene Abschnitt der mindestens einen Anschlussleitung kann mit dem mindestens einen daran festgelegten oder festlegbaren Verbindungselement am Instrumentengriff des gebildeten Rohrschaftinstrumentes festgelegt werden, so dass die Handhabung des Rohrschaftinstrumentes verbessert wird. Die lösbare Verbindung der mindestens einen Anschlussleitung mit dem Instrumentengriff erlaubt es auch, ein Wechselrohrschaftsystem bereitzustellen, bei dem ein wiederverwendbarer Instrumentengriff mit mehreren Rohrschäften zum Einsatz kommen kann. Beim jeweiligen Einsatz eines Rohrschaftes wird dessen mindestens eine Anschlussleitung mit dem Instrumentengriff verbunden und nach dem Einsatz wieder von diesem gelöst, so dass der Rohrschaft auf benutzerfreundliche Weise entfernt und entsorgt werden kann.

Das mindestens eine Verbindungselement kann ein Klemmelement und/oder ein Rastelement sein, es kann form- und/oder kraftschlüssig am Instrumentengriff festlegbar sein. Denkbar ist auch, dass das mindestens eine Verbindungselement durch Verschraubung am Instrumentengriff festlegbar ist.

Vorzugsweise kann das mindestens eine Verbindungselement werkzeuglos mit dem Instrumentengriff verbunden und/oder von diesem gelöst werden. Das mindestens eine Verbindungselement wirkt vorzugsweise mit einem korrespondierend dazu ausgebildeten Verbindungselement des Instrumentengriffes zusammen, so dass die Verbindungselemente eine Verbindungseinrichtung zum lösbaren Festlegen der mindestens einen Anschlussleitung am Instrumentengriff ausbilden.

Günstig ist es, wenn das Anschlusselement ein Verbindungselement umfasst oder ausbildet. Dies ermöglicht eine konstruktiv einfache Ausgestaltung des Rohrschaftes. Insbesondere ist es von Vorteil, wenn außer dem vom Anschlusselement umfassten oder ausgebildeten Verbindungselement kein weiteres Verbindungselement des Rohrschafts vorgesehen ist.

Vorzugsweise ist das Verbindungselement einstückig mit dem Anschlusselement gebildet, um eine konstruktiv einfache Ausgestaltung des Rohrschaftes zu ermöglichen.

Als vorteilhaft erweist es sich, wenn das mindestens eine Verbindungselement einen Vorsprung umfasst, der in eine korrespondierende Aufnahme am Instrumentengriff einführbar ist, wobei die Aufnahme ein korrespondierend zum Vorsprung ausgebildetes Verbindungselement des Instrumentengriffes ist. Der Vorsprung kann beispielsweise mit der Aufnahme verklemmen und/oder verrasten, um eine kraft- und/oder formschlüssige Verbindung des Verbindungselementes mit dem Instrumentengriff zu ermöglichen. Auch eine Verschraubung von Vorsprung und Aufnahme ist möglich.

Alternativ oder ergänzend kann vorgesehen sein, dass das mindestens eine Verbindungselement eine Aufnahme am Anschlusselement umfasst, in der ein korrespondierender Vorsprung am Instrumentengriff einführbar ist.

Der Vorsprung kann beispielsweise distalseitig am mindestens einen Verbindungselement angeordnet sein, insbesondere an einer distalen Stirnseite des Verbindungselementes. Dies erlaubt es, dem Rohrschaftinstrument einen kompakten Aufbau zu verleihen, wenn proximalseitig am Instrumentengriff, beispielsweise an einer proximalen Stirnseite, ein korrespondierend zum Vorsprung ausgebildetes Verbindungselement angeordnet ist.

Der Vorsprung kann insbesondere als Rippe oder Leiste ausgestaltet sein.

Von Vorteil ist es, wenn der Vorsprung eine Achse definiert, die im Winkel zu einer vom Rohr definierten proximal-distalen Achse ausgerichtet ist. Die vom Vorsprung definierte Achse kann eine Einführrichtung zu dessen Einführen in die Aufnahme am Instrumentengriff definieren. Durch den Winkel zwischen der Achse des Vorsprungs und der proximal-distalen Achse kann beispielsweise sichergestellt werden, dass der Vorsprung nicht axial längs der proximal-distalen Achse in die Aufnahme eingeführt wird (oder parallel dazu). Dies erlaubt es, beim Einführen des Vorsprungs in die Aufnahme die Zugkraft auf die mindestens eine Anschlussleitung zu verringern und dadurch deren mögliche Beschädigung zu vermeiden.

Vorteilhafterweise ist der Winkel zwischen den Achsen ein spitzer Winkel, er kann beispielsweise ungefähr 70° bis ungefähr 85° betragen, zum Beispiel ungefähr 80°. Ein beim Einführen des Vorsprungs in die Aufnahme führendes Ende des Vorsprungs ist günstigerweise distaler angeordnet als ein Ende des Vorsprungs, das zuletzt in die Aufnahme eingeführt wird, so dass zwischen dem mindestens einen Verbindungselement des Rohrschaftes und dem korrespondierenden Verbindungselement am Instrumentengriff ein Hinterschnitt gebildet werden kann. Der spitze Winkel zwischen den Achsen kann damit auf eine proximal-distale Richtung und eine Einführrichtung des Vorsprungs in die Aufnahme am Instrumentengriff bezogen aufgefasst werden.

Günstig ist es, wenn das mindestens eine Verbindungselement ein Anlageglied für den Instrumentengriff umfasst oder ausbildet, insbesondere an einer distalen Stirnseite. Dadurch kann eine zuverlässige Relativpositionierung des mindestens einen Verbindungselementes und des Instrumentengriffes sichergestellt werden. Beispielsweise kann das mindestens eine Verbindungselement flanschartig über das Anlageglied am Instrumentengriff anliegen. Das Anlageglied kann planar ausgestaltet sein, und es kann eine Ebene definieren, parallel zu der die vorstehend erwähnte vom Vorsprung definierte Achse verläuft. Die Ebene kann relativ zu einer proximal-distalen Achse geneigt sein. Vom Anlageglied kann der vorstehend erwähnte Vorsprung in distaler Richtung vorspringen.

Als günstig erweist es sich, wenn das mindestens eine Verbindungselement eine zylindrische oder im Wesentlichen zylindrische Außenkontur aufweist, insbesondere in Umfangsrichtung einer vom Rohr definierten proximal-distalen Achse. Das mindestens eine Verbindungselement ist beispielsweise zylindrisch und insbesondere scheibenförmig ausgestaltet mit einer zylindrischen Außenkontur, die günstigerweise an eine Außenkontur des Instrumentengriffes angepasst ist. Dies erlaubt es, dem so gebildeten Rohrschaftinstrument ein gefälligeres Aussehen zu verleihen und vereinfacht dessen Handhabung.

Wie eingangs erwähnt, betrifft die Erfindung auch einen chirurgischen Instrumentengriff. Bei einem gattungsgemäßen Instrumentengriff wird die eingangs genannte Aufgabe erfindungsgemäß dadurch gelöst, dass der Instrumentengriff für die Verwendung mit einem Rohrschaft der vorstehend genannten Art ausgebildet ist und mindestens ein Verbindungselement zum lösbaren Festlegen der mindestens einen Anschlussleitung des Rohrschaftes am Instrumentengriff umfasst, wobei das mindestens eine Verbindungselement proximalseitig am Instrumentengriff angeordnet ist, insbesondere an einer proximalen Stirnseite des Instrumentengriffes, und dass der Instrumentengriff frei von innenliegenden elektrischen Kontaktgliedern zur elektrischen Kontaktierung des elektrochirurgischen Rohrschaftes ist.

Die unter Einsatz des erfindungsgemäßen Rohrschaftes oder vorteilhafter Ausführungsformen davon erzielbaren Vorteile könnten mit dem erfindungsgemäßen Instrumentengriff ebenfalls erzielt werden, so dass diesbezüglich auf voranstehende Erläuterungen verwiesen werden kann. Insbesondere ist die mindestens eine Anschlussleitung des Rohrschafts proximalseitig am Instrumentengriff lösbar festlegbar, der zu diesem Zweck mindestens ein Verbindungselement umfasst. Das mindestens eine Verbindungselement kann insbesondere mit dem vorstehend erwähnten mindestens einen Verbindungselement, das an dem aus dem Rohr ausgetretenen Abschnitt der Anschlussleitung festgelegt oder festlegbar ist, zusammenwirken und mit diesem eine Verbindungseinrichtung ausbilden. Das mindestens eine Verbindungselement des Instrumentengriffes kann zur klemmenden und/oder rastenden, zur form- und/oder kraftschlüssigen Verbindung ausgebildet sein. Ebenso ist eine werkzeuglose Verbindung und/oder werkzeugloses Lösen der mindestens einen Anschlussleitung mit dem bzw. vom Instrumentengriff möglich. Denkbar ist auch eine Verschraubung der Verbindungselemente miteinander.

Bei einer konstruktiv einfachen Ausgestaltung kann das mindestens eine Verbindungselement beispielsweise eine Aufnahme umfassen oder ausbilden, in die die mindestens eine elektrische Anschlussleitung einführbar ist. Beispielsweise kann diese in der Aufnahme verklemmt werden.

Von Vorteil ist es, wenn das mindestens eine Verbindungselement eine Aufnahme umfasst oder ausbildet, in die ein an der mindestens einen elektrischen Anschlussleitung angeordneter Vorsprung einführbar ist, insbesondere zur kraft- und/oder formschlüssigen Fixierung des Vorsprungs in der Aufnahme.

Alternativ oder ergänzend kann das mindestens eine Verbindungselement einen Vorsprung aufweisen, der in eine korrespondierend dazu ausgebildete Aufnahme eines Verbindungselementes des Rohrschaftes einführbar ist.

Bevorzugt ist die Aufnahme sacklochförmig. Dadurch ist die Möglichkeit gegeben, den Vorsprung in nur einer Einführrichtung in die Aufnahme einzuführen. Dies erleichtert die Handhabung des Instrumentengriffes und hilft, ein fehlerhaftes Verbinden der mindestens einen Anschlussleitung mit dem Instrumentengriff zu vermeiden.

Von Vorteil ist es, wenn die Aufnahme eine Achse definiert, die im Winkel zu einer vom Instrumentengriff definierten proximal-distalen Achse ausgerichtet ist. Wie bereits erwähnt, erlaubt die Ausrichtung der im Winkel zueinander stehenden Achsen, den Vorsprung nicht in rein axialer Richtung in die Aufnahme einzuführen. Die Zugkraft auf die mindestens eine Anschlussleitung kann dadurch möglichst gering gehalten werden.

Der Winkel zwischen den Achsen ist bevorzugt ein spitzer Winkel und kann beispielsweise ungefähr 70° bis ungefähr 85° betragen, insbesondere ungefähr 80°. Die Aufnahme umfasst bevorzugt eine Einführöffnung zum Einführen des Vorsprungs, die proximaler angeordnet ist als ein der Einführöffnung gegenüberliegendes Ende der Aufnahme, beispielsweise ein Boden des Sackloches. Der Vorsprung kann dadurch gewissermaßen von proximal nach distal schief zur distal-proximalen Achse in die Aufnahme eingeführt werden, um einen Hinterschnitt zwischen dem Vorsprung und der Aufnahme zu bilden und dadurch eine zuverlässige Fixierung der mindestens einen Anschlussleitung am Instrumentengriff zu ermöglichen.

Bei einer andersartigen Ausgestaltung der Verbindungselemente ist denkbar, dass das Anschlusselement mit dem Instrumentengriff axial längs einer vom Instrumentengriff definierten Griffachse verbunden werden kann. Der Vorsprung und die entsprechende Aufnahme können axial ineinander eingeführt und insbesondere klemmend und/oder rastend miteinander verbunden werden. Denkbar ist auch eine Verschraubung des Vorsprungs in der Aufnahme oder eine bajonettartige Verbindung, wobei das Anschlusselement relativ zum Instrumentengriff gedreht oder geschwenkt wird, um diese miteinander zu verbinden.

Bei einer vorteilhaften Ausführungsform des Instrumentengriffes umfasst das Verbindungselement einen Vorsprung, der als Luer-Anschluss ausgestaltet ist, insbesondere für eine Luer-Lock-Verbindung mit dem Anschlusselement des Rohrschaftes. Das Anschlusselement kann durch Verschraubung mit dem Instrumentengriff verbunden werden, wobei der Luer-Vorsprung am Instrumentengriff in eine Luer-Aufnahme am Anschlusselement eingreift.

Der Luer-Anschluss ist bevorzugt zum Anschluss einer Spülleitung ausgestaltet, so dass der Instrumentengriff von proximal mit einer Spülflüssigkeit beaufschlagt und gereinigt werden kann. Auf den Anschluss einer Spülleitung wird nachfolgend noch eingegangen.

Als günstig erweist es sich, wenn das mindestens eine Verbindungselement ein Anlageglied für ein Verbindungselement des Rohrschaftes umfasst oder ausbildet, insbesondere an einer proximalen Stirnseite des Instrumentengriffes. Das Anlageglied ist beispielsweise planar ausgestaltet, und es kann eine im Winkel relativ zur proximal-distalen Richtung ausgerichtete Ebene definieren. Das Anlageglied kann beispielsweise für eine flanschartige Anlage am mindestens einen Verbindungselement des Rohrschaftes ausgebildet sein. Die Anordnung des mindestens einen Anlagegliedes an einer proximalen Stirnseite des Instrumentengriffes ermöglicht eine kompakte Bauform desselben.

Von Vorteil ist es, wenn der Instrumentengriff einen hülsenförmigen Grundkörper umfasst sowie ein proximalseitig mit dem Grundkörper verbundenes Verschlusselement zum Verschließen des Grundkörpers. In den hülsenförmigen Grundkörper kann der Rohrschaft von distal zumindest teilweise eingeführt werden und dadurch mit dem Instrumentengriff koppeln. Proximalseitig ist das Verschlusselement vorgesehen, um den Grundkörper proximalseitig zu schützen. Das Verschlusselement kann den Grundkörper dichtend oder nicht dichtend verschließen. Es kann insbesondere vorgesehen sein, dass ein Medienaustausch zwischen dem Inneren des Grundkörpers und der Umgebung durch das Verschlusselement hindurch möglich ist. Beispielsweise ist das Verschlusselement stopfenförmig, stöpselförmig oder kappenförmig ausgestaltet.

Vorzugsweise weist das Verschlusselement einen Durchgangskanal in proximal-distaler Richtung auf. Dies erlaubt es, proximal eine Spülleitung an den Instrumentengriff anzuschließen und eine Spülflüssigkeit zum Reinigen desselben durch das Verschlusselement in den Grundkörper einzuleiten.

Vorteilhafterweise umfasst das Verschlusselement das mindestens eine Verbindungselement oder bildet dieses aus, um eine konstruktiv einfache Ausgestaltung des Instrumentengriffes zu ermöglichen. Beispielsweise umfasst das Verbindungselement den vorstehend erwähnten Luer-Vorsprung zum Anschließen einer Spülleitung.

Bevorzugt ist das mindestens eine Verbindungselement einstückig mit dem Verschlusselement gebildet, ebenfalls zur Erzielung einer konstruktiv einfachen Ausgestaltung.

Günstig ist es, wenn das Verschlusselement lösbar mit dem Grundkörper verbindbar ist. Dies gibt die Möglichkeit, das Verschlusselement beispielsweise bei Bedarf gegen ein anderes Verschlusselement auszutauschen, welches etwa keinen Durchgangskanal und/oder kein Verbindungselement umfasst oder ausbildet. Es ist auch denkbar, ein einen Durchgangskanal und/oder ein Verbindungselement umfassendes oder ausbildendes Verschlusselement separat vorzuhalten und mit einem Instrumentengriff zu verbinden, so dass die vorstehend erläuterten Funktionen und Vorteile mit dem Instrumentengriff erzielt werden können.

Es kann vorgesehen sein, dass das mindestens eine Verbindungselement eine zylindrische oder im Wesentlichen zylindrische Außenkontur aufweist, insbesondere in Umfangsrichtung einer vom Instrumentengriff definierten proximal-distalen Achse. Die Außenkontur des Verbindungselementes des Instrumentengriffes kann insbesondere an die Außenkontur eines Verbindungselementes des Rohrschaftes angepasst sein.

Bei einer vorteilhaften Ausführungsform des erfindungsgemäßen Instrumentengriffes ist der Instrumentengriff ein Axialgriff und weist einen hülsenförmigen Grundkörper auf sowie mindestens ein betätigbares Griffelement, das relativ zum Grundkörper von einer abgespreizten Stellung in eine angenäherte Stellung überführbar ist. Das mindestens eine Griffelement ist beispielsweise schwenkbar am Grundkörper gelagert, und es kann in der abgespreizten Stellung eine Nichtbetätigungsstellung einnehmen und in der angenäherten Stellung eine Betätigungsstellung. Durch Betätigen des Griffelementes kann auf das Werkzeug eines mit dem Instrumentengriff gekoppelten Rohrschaftes eingewirkt und dieses betätigt werden. Das mindestens eine Griffelement ist beispielsweise halbschalenförmig oder im Wesentlichen halbschalenförmig ausgestaltet und kann den Grundkörper in der angenäherten Stellung in Umfangsrichtung abschnittsweise umgeben. Es können insbesondere zwei betätigbare Griffelemente vorgesehen sein.

Wie eingangs erwähnt betrifft die Erfindung auch ein elektrochirurgisches Rohrschaftinstrument. Ein erfindungsgemäßes Rohrschaftinstrument, das die eingangs genannte Aufgabe löst, umfasst die Merkmale des unabhängigen Anspruches 12.

Unter Einsatz des erfindungsgemäßen Rohrschaftinstrumentes können die bereits im Zusammenhang mit dem erfindungsgemäßen Rohrschaft und vorteilhafter Ausführungsformen davon bzw. die bereits im Zusammenhang mit dem erfindungsgemäßen Instrumentengriff und vorteilhafter Ausführungsformen davon erzielbaren Vorteile ebenfalls erzielt werden. Diesbezüglich wird auf voranstehende Erläuterungen verwiesen.

Die Verbindungselemente bilden eine Verbindungseinrichtung zur kraft- und/oder formschlüssigen, zur klemmenden und/oder rastenden und/oder werkzeuglosen Befestigung der mindestens einen Anschlussleitung am Instrumentengriff. Denkbar ist auch die Ausbildung einer Schraubverbindung oder einer bajonettartigen Verbindung.

Der Instrumentengriff kann eine proximal-distale Achse definieren, die mit einer vom Rohr des Rohrschafts definierten proximal-distalen Achse in Übereinstimmung gebracht werden kann, wenn der Instrumentengriff und der Rohrschaft aneinander angekoppelt sind. Die von dieser Achse definierte Richtung wird nachfolgend als axiale Richtung bezeichnet.

Günstig ist es, wenn die mindestens eine Anschlussleitung so bemessen ist, dass sie mit ihrem aus dem Rohr ausgetretenen Abschnitt frei oder im Wesentlichen frei von axialen Zug- und/oder Schubkräften ist. Dadurch ist insbesondere die Möglichkeit gegeben, die mindestens eine Anschlussleitung zugentlastet oder im Wesentlichen zugentlastet mit dem Instrumentengriff zu verbinden. Die mindestens eine Anschlussleitung unterliegt dadurch nur geringen Belastungen, so dass deren Beschädigung weitgehend vermieden werden kann.

Eine kompakte Bauform des Rohrschaftinstrumentes kann zum Beispiel dadurch erzielt werden, dass der aus dem Rohr ausgetretene Abschnitt der mindestens einen Anschlussleitung längs des Instrumentengriffes achsparallel distal-proximal verläuft, zumindest abschnittsweise.

Der aus dem Rohr ausgetretene Abschnitt verläuft insbesondere, zumindest abschnittsweise, parallel zum Grundkörper des Instrumentengriffes. Es kann vorgesehen sein, dass der Abschnitt oder eine den Abschnitt umgebende Umhüllung am Grundkörper anliegt.

Vorteilhaft ist es, wenn der aus dem Rohr ausgetretene Abschnitt der mindestens einen Anschlussleitung quer zur Axialrichtung seitlich neben einem betätigbaren Griffelement des Instrumentengriffes zum Einwirken auf das Werkzeug verläuft. Dadurch kann sichergestellt werden, dass die Betätigung des Griffelementes durch den Abschnitt der mindestens einen Anschlussleitung nicht beeinträchtigt wird. Günstigerweise ist vorgesehen, dass das Griffelement den Abschnitt der mindestens einen Anschlussleitung beim Überführen von einer Nichtbetätigungsstellung in eine Betätigungsstellung nicht kontaktiert.

Wie bereits erwähnt, kann vorgesehen, dass der Instrumentengriff zwei betätigbare Griffelemente zum Einwirken auf das Werkzeug umfasst. Hierbei ist günstigerweise vorgesehen, dass zwischen den Griffelementen, bezogen auf eine Richtung quer zur Axialrichtung, der aus dem Rohr ausgetretene Abschnitt der mindestens einen Anschlussleitung verläuft. Dadurch kann eine kompakte Bauform des Rohrschaftinstrumentes erzielt werden.

Es wurde ebenfalls bereits erwähnt, dass die Außenkonturen des mindestens einen Verbindungselementes des Rohrschaftes und des mindestens einen Verbindungselementes des Instrumentengriffes aneinander angepasst sein können. Im miteinander verbundenen Zustand können die Verbindungselemente eine quasi kontinuierliche gemeinsame Außenkontur aufweisen. Dies verleiht dem Rohrschaftinstrument ein gefälligeres Aussehen und erleichtert dessen Handhabung.

Beispielsweise kann vorgesehen sein, dass die Verbindungselemente jeweils, insbesondere in Umfangsrichtung einer vom Instrumentengriff definierten proximal-distalen Achse, eine zylindrische oder im Wesentlichen zylindrische Außenkontur gleichen Durchmessers aufweisen.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine bevorzugte Ausführungsform eines erfindungsgemäßen Rohrschaftinstrumentes in perspektivischer Ansicht, umfassend eine bevorzugte Ausführungsform eines erfindungsgemäßen Rohrschaftes und eine bevorzugte Ausführungsform eines erfindungsgemäßen Instrumentengriffes, wobei der Rohrschaft und der Instrumentengriff im noch nicht miteinander verbundenen Zustand gezeigt sind;
- Figur 2:: eine vergrößerte Darstellung eines proximalen Abschnittes des Rohrschaftinstrumentes aus Figur 1, wobei der Rohrschaft und der Instrumentengriff miteinander verbunden sind;

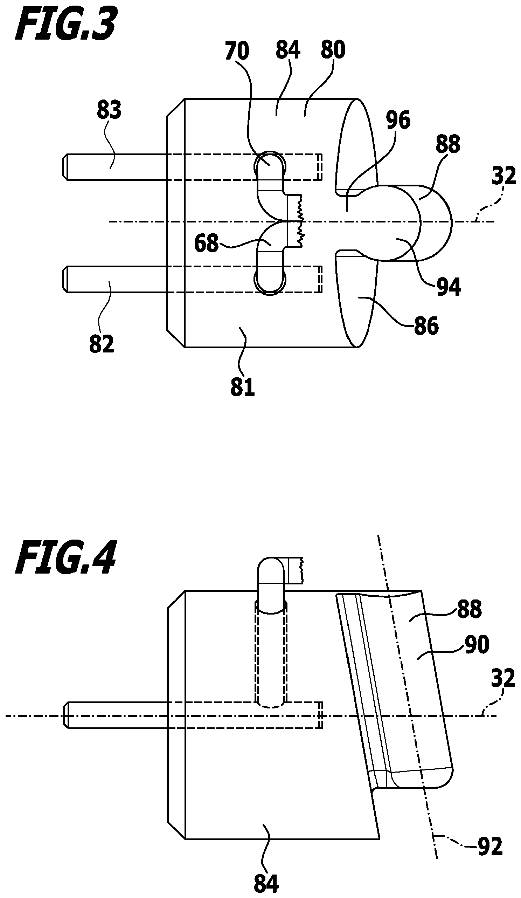

- Figur 3:: eine Draufsicht auf ein Anschlusselement des Rohrschafts aus Figur 1;
- Figur 4:: eine Seitenansicht des Anschlusselementes aus Figur 3;
- Figur 5:: eine Draufsicht auf ein Verschlusselement des Instrumentengriffs aus Figur 1;
- Figur 6:: eine Schnittansicht längs der Linie 6-6 in Figur 5;
- Figur 7:: eine teilweise Darstellung eines proximalen Endbereiches des Rohrschaftinstrumentes aus Figur 1, wobei das Anschlusselement aus Figur 3 mit dem Verschlusselement aus Figur 5 verbunden wird;
- Figur 8:: eine abschnittsweise Darstellung eines distalen Endbereiches des Instrumentengriffes und des proximalen Endbereiches des Rohrschafts aus Figur 1, teilweise geschnitten;
- Figur 9:: eine weitere bevorzugte Ausführungsform eines erfindungsgemäßen Rohrschaftinstrumentes in perspektivischer Ansicht, umfassend eine bevorzugte Ausführungsform eines erfindungsgemäßen Rohrschaftes und eine bevorzugte Ausführungsform eines erfindungsgemäßen Instrumentengriffes, wobei der Rohrschaft und der Instrumentengriff im miteinander verbundenen Zustand gezeigt sind;
- Figur 10:: eine vergrößerte Darstellung eines proximalen Abschnittes des Rohrschaftinstrumentes aus Figur 9 in einer Explosionsdarstellung und
- Figur 11:: nochmals den proximalen Abschnitt aus Figur 10 in einer anderen Perspektive.

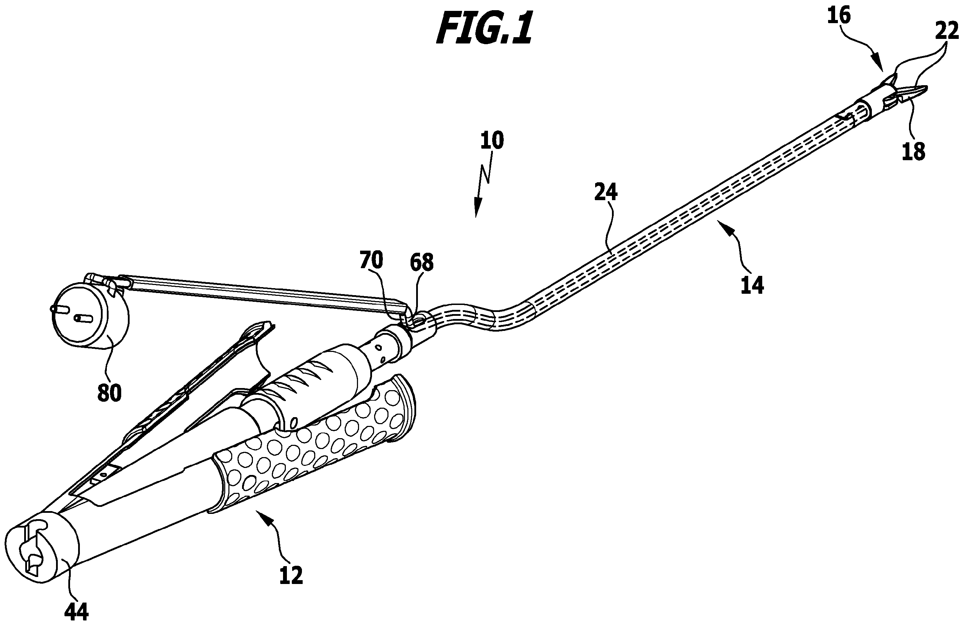

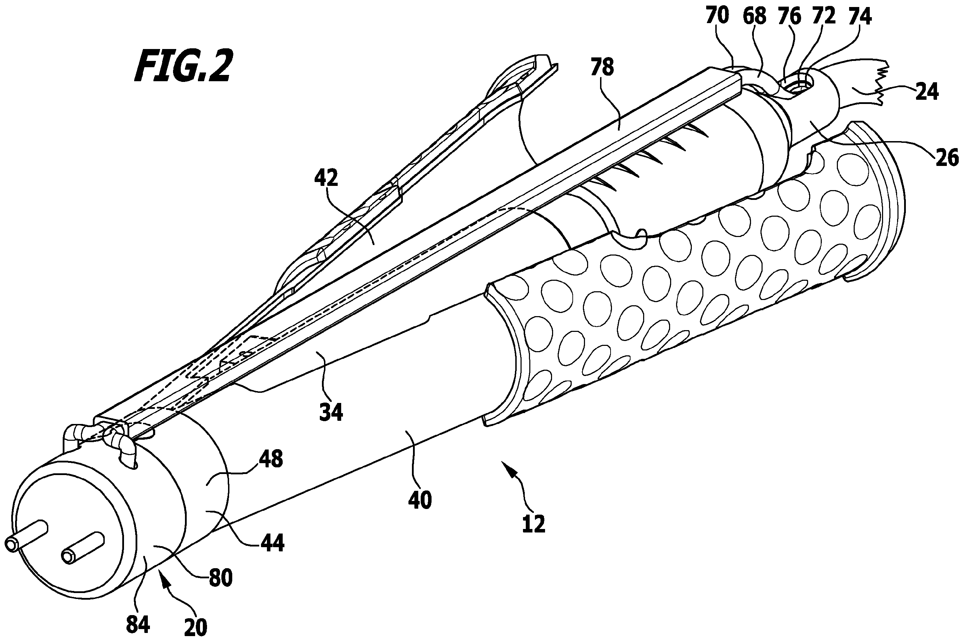

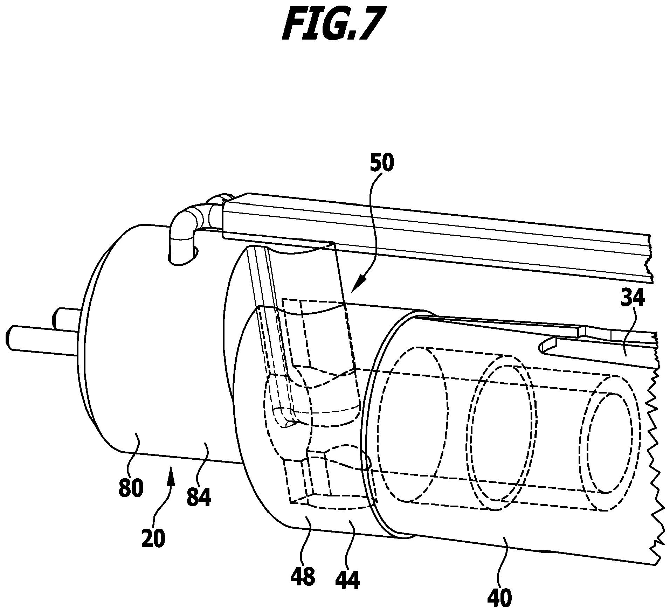

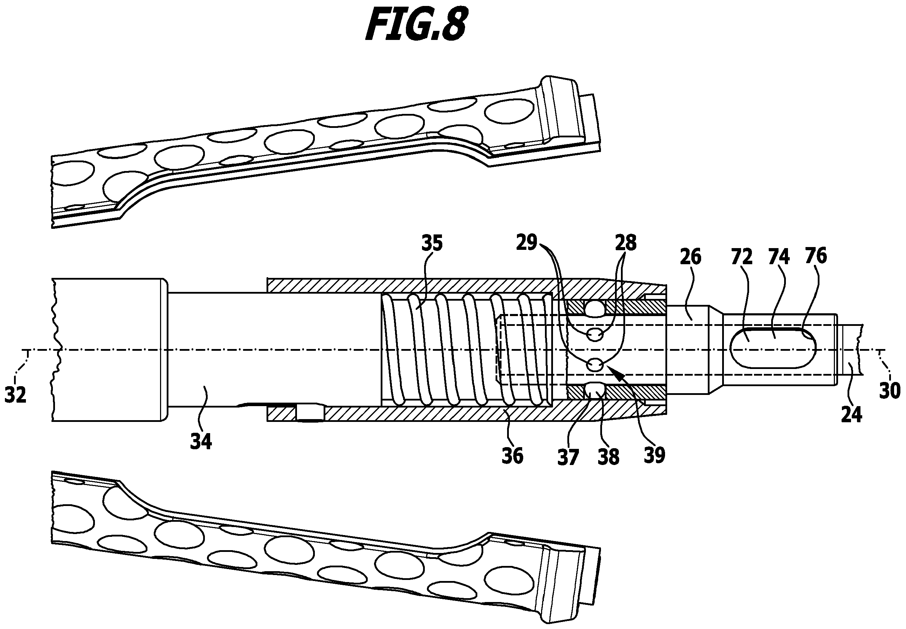

Figur 1 zeigt in perspektivischer Darstellung eine bevorzugte Ausführungsform eines insgesamt mit dem Bezugszeichen 10 belegten erfindungsgemäßen elektrochirurgischen Rohrschaftinstrumentes. Das Rohrschaftinstrument 10 umfasst eine bevorzugte Ausführungsform eines mit dem Bezugszeichen 12 belegten erfindungsgemäßen chirurgischen Instrumentengriffes und eine bevorzugte Ausführungsform eines mit dem Bezugszeichen 14 belegten erfindungsgemäßen elektrochirurgischen Rohrschaftes. Der Instrumentengriff 12 und der Rohrschaft 14 sind lösbar miteinander verbindbar und in den Figuren 1 und 7 (teilweise) dargestellt, während sie miteinander verbunden werden, wohingegen der Instrumentengriff 12 und der Rohrschaft 14 in den Figuren 2 und 8 (jeweils teilweise) im miteinander verbundenen Zustand dargestellt sind.

Positions- und Orientierungsangaben wie "proximal", "distal", "proximal-distal" und dergleichen sind vorliegend auf einen bestimmungsgemäßen Gebrauch des Rohrschaftinstrumentes 10 bezogen aufzufassen, in dem der Instrumentengriff 12 und der Rohrschaft 14 miteinander verbunden sind. Im bestimmungsgemäßen Gebrauch wirkt der Benutzer von proximal auf das Rohrschaftinstrument 10 ein und operiert mit einem am distalen Ende 16 des Rohrschaftinstrumentes 10 angeordneten Werkzeug 18. Das proximale Ende des Rohrschaftinstrumentes 10 ist mit dem Bezugszeichen 20 belegt.

Der Rohrschaft 14 ist ein bipolarer elektrochirurgischer Rohrschaft und umfasst distalseitig das bereits erwähnte Werkzeug 18, das ausgestaltet ist als Pinzettenwerkzeug mit zwei relativ zueinander beweglichen und insbesondere verschwenkbaren Maulteilen 22. Die Maulteile 22 können von einer geöffneten Stellung (Figur 1), in der sie im Abstand zueinander angeordnet sind, in eine geschlossene Stellung (nicht gezeigt) überführt werden, so dass zu koagulierendes Körpergewebe zwischen ihnen gefasst werden kann.

Das Werkzeug 18 ist am distalen Ende eines Rohres 24 des Rohrschafts 14 angeordnet, das zwei parallel zueinander versetzte Abschnitte distalseitig und proximalseitig umfasst, die durch zweifache Krümmung des Rohres 24 ineinander übergehen. Das Rohr 24 könnte jedoch auch geradlinig ausgebildet sein oder eine andersartige Krümmung aufweisen.

Der proximalseitige Abschnitt des Rohres 24 ist formschlüssig in einer Buchse 26 des Rohrschafts 14 aufgenommen. Proximal an der Buchse 26, und damit proximal am Rohrschaft 14, sind Kopplungselemente 28 in Gestalt von konkaven Ausnehmungen 28 vorhanden. Der proximale Abschnitt des Rohres 24 definiert eine proximal-distale Rohrachse 30. Die Rohrachse 30 kann mit einer vom Instrumentengriff 12 definierten Griffachse 32 in Übereinstimmung gebracht werden, wenn der Rohrschaft 14 und der Instrumentengriff bestimmungsgemäß miteinander verbunden sind. Bezugnahmen auf die Griffachse 32 sind nachfolgend daher gleichzeitig Bezugnahmen auf die Rohrachse 30 und umgekehrt.

Im Rohr 24 ist ein an sich bekanntes und in der Zeichnung nicht dargestelltes Zug- und/oder Schubkraftübertragungselement geführt, das distal mit den Maulteilen 22 in Wirkverbindung steht, proximalseitig aus dem Rohr 24 austritt und ein Kopplungselement zum Ankoppeln an den Instrumentengriff 12 umfasst.

Der Instrumentengriff 12 ist als Axialgriff ausgestaltet und umfasst einen im Wesentlichen hohlzylindrischen hülsenförmigen Grundkörper 34, der die Griffachse 32 definiert. Distalseitig weist der Instrumentengriff 12 eine auf dem Grundkörper 34 gegen die Kraft eines elastischen Rückstellelementes 35, ausgestaltet als Schraubenfeder, verschieblich gelagerte Hülse 36 auf. Die Hülse 36 umfasst innenseitig Kopplungselemente 37 in Gestalt von Rastkugeln 38. Beim Einführen der Buchse 26 in die Hülse 36 koppeln die Ausnehmungen 29 mit den Rastkugeln 38, die miteinander eine Kopplungseinrichtung 39 des Rohrschaftinstrumentes ausbilden, so dass die Buchse 26, und damit der Rohrschaft 14, lösbar am Instrumentengriff 12 festgelegt werden kann.

Eine weitere Kopplungseinrichtung, die in der Zeichnung nicht dargestellt ist, wird gebildet durch das in der Zeichnung nicht dargestellte Kopplungselement des Zug- und/oder Schubkraftübertragungselementes des Rohrschaftes 14. Dieses Kopplungselement koppelt mit einem in der Zeichnung ebenfalls nicht dargestellten Kopplungselement des Instrumentengriffes 12 in Gestalt eines im Grundkörper 34 axial verschieblich gelagerten Zug- und/oder Schubgliedes, das an sich bekannt ist.

Zum Einwirken auf das Zug- und/oder Schubglied umfasst der Instrumentengriff 12 zwei Griffelemente 40 und 42, die in an sich bekannter Weise am proximalen Ende des Grundkörpers 34 um quer zur Griffachse 32 verlaufende Schwenkachsen schwenkbar gelagert sind. "Quer zur Griffachse 32" bedeutet vorliegend in einer senkrecht zur Griffachse 32 ausgerichteten Ebene. Die Griffelemente 40 und 42 liegen einander bezüglich der Griffachse 32 diametral gegenüber und sind näherungsweise halbschalenförmig ausgestaltet. Von einer abgespreizten Stellung (Figuren 1, 2 und 8) können die Griffelemente 40 und 42 relativ zum Grundkörper 34 verschwenkt und in Bezug auf diesen in eine angenäherte Stellung überführt werden (nicht gezeigt). Dabei wirken die Griffelemente 40 und 42 in an sich bekannter Weise auf das Zug- und/oder Schubglied ein, so dass mittels des Zug- und/oder Schubkraftübertragungselementes auf die Maulteile 22 zu deren Betätigung eingewirkt werden kann.

Am proximalen Ende umfasst der Instrumentengriff 12 ein Verschlusselement 44, das zum teilweisen Verschließen des Grundkörpers 34 von proximal in diesen eingeführt ist. Das Verschlusselement 44 ist vorliegend stopfenförmig ausgestaltet, es könnte jedoch auch eine kappen- oder stöpselförmige Gestalt aufweisen. Im Verschlusselement 44 ist ein axialer Durchgangskanal 46 gebildet. Dadurch ist die Möglichkeit gegeben, eine Leitung für Reinigungs- oder Spülflüssigkeit von proximal an das Verschlusselement 44 anzuschließen und durch dieses hindurch den wiederverwendbaren Instrumentengriff 12 nach dem Gebrauch und dem Lösen des Rohrschaftes 14 zu reinigen. Das Verschlusselement 44 übergreift proximalseitige, in der Zeichnung nicht dargestellte Lagerglieder der Griffelemente 40 und 42, über welche die Griffelemente 40 und 42 am Grundkörper 34 schwenkbar gelagert sind.

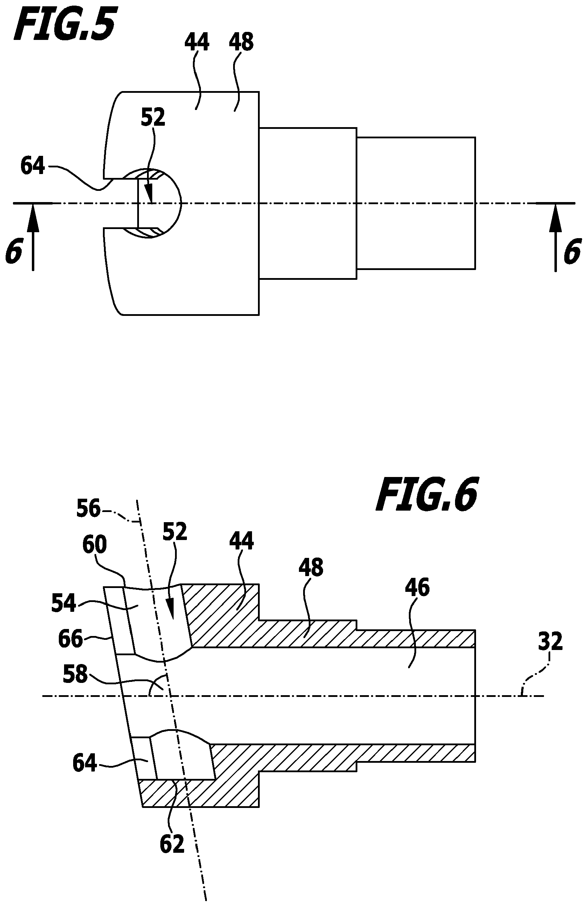

Das Verschlusselement 44 ist einstückig gebildet und bildet ein Verbindungselement 48 aus, das mit einem korrespondierend dazu ausgebildeten Verbindungselement des Rohrschafts 14, auf dieses wird nachfolgend noch eingegangen, zur Ausbildung einer Verbindungseinrichtung 50 des Rohrschaftinstrumentes 10 zusammenwirken kann. Zu diesem Zweck umfasst das Verschlusselement 44 proximalseitig eine Aufnahme 52, die ausgestaltet ist als Sackloch 54. Das Sackloch 54 ist geradlinig erstreckt im Verschlusselement 44 gebildet und definiert eine Lochachse 56, die einen Winkel 58 mit der Griffachse 32 einschließt. Der Winkel 58 ist ein schiefer Winkel, bezogen auf eine proximal-distale Richtung, wobei eine Einführöffnung 60 des Sacklochs 54 proximaler am Instrumentengriff 12 angeordnet ist als ein der Einführöffnung 60 gegenüberliegendes Ende des Sackloches 54, das mit einem Boden 62 verschlossen ist (Figuren 5 und 6). Die Einführöffnung 60 ist am Außenumfang des Verschlusselementes angeordnet. Der Durchgangskanal 46 und die Aufnahme 52 schneiden einander ebenfalls unter dem Winkel 58.

Das Verschlusselement 44 ist proximalseitig mit einer schlitzförmigen Durchbrechung 64 versehen, die in der proximalseitigen Wand der Aufnahme 52 gebildet ist. Quer zu einer von der Griffachse 32 und der Lochachse 56 definierten Ebene ist die Durchbrechung 64 schmaler als das im Querschnitt runde Sackloch 54. Hiervon ausgenommen ist der Abschnitt der Aufnahme 52, der vom axialen Durchgangskanal 46 durchsetzt ist.

An der proximalen Stirnseite ist das Verschlusselement 44 planar ausgestaltet und weist ein relativ zur Griffachse 32 geneigtes Anlageglied 66 auf. Eine vom Anlageglied 66 definierte Ebene verläuft parallel zur Lochachse 46 und schneidet die Griffachse 32 ebenfalls unter dem Winkel 58.

Wie bereits erwähnt, ist der Rohrschaft 14 ein bipolarer Rohrschaft. Zur elektrischen Kontaktierung umfasst der Rohrschaft 14 zwei elektrische Anschlussleitungen 68 und 70. Die Anschlussleitungen 68 und 70 stehen mit je einem der Maulteile 22 in elektrischer Verbindung, so dass diese auf entgegengesetzte elektrische Polarität gebracht werden können, um in der Schließstellung zwischen den Maulteilen 22 gefasstes Gewebe zu koagulieren.

Die Anschlussleitungen 68 und 70 sind als flexible, elektrisch isolierte Kabelleitungen ausgestaltet, die ausgehend vom distalen Ende 16 im Rohr 24 bis zur Buchse 26 verlaufen. Auf diese Weise sind die Anschlussleitungen 68 und 70 fern vom Operationsfeld gehalten, was die Handhabung des Rohrschaftinstrumentes 10 erleichtert und dessen elektrische Sicherheit erhöht.

Wie insbesondere in Figur 2 zu erkennen ist, weist das Rohr 24, bezogen auf die Rohrachse 30, eine seitliche Austrittsöffnung 72 in Gestalt einer Durchbrechung 74 auf. Die Durchbrechung 74 ist distal der Kopplungselemente 28 am proximalen Abschnitt des Rohres 24 angeordnet, der von der Buchse 26 umgeben ist. In der Buchse 26 ist eine mit der Durchbrechung 74 fluchtende Durchbrechung 76 gebildet. Im verbundenen Zustand des Rohrschaftes 14 und des Instrumentengriffes 12 sind die Durchbrechungen 74 und 76 im Wesentlichen unmittelbar distal des Instrumentengriffes 12 angeordnet, an dem nicht in die Hülse 36 eingreifenden Abschnitt der Buchse 26 bzw. des Rohres 24.

Die Anschlussleitungen 68 und 70 sind durch die Durchbrechungen 74 und 76 hindurchgeführt, so dass sie aus dem Rohr 24 distal des Instrumentengriffes 12 austreten und mit ihren proximal bezüglich der Durchbrechungen 74 und 76 angeordneten Abschnitten außerhalb des Instrumentengriffes 12 verlaufen. Dies erlaubt eine elektrische Kontaktierung des Rohrschaftes 14, ohne dass zu diesem Zweck im Instrumentengriff 12, anders als bei gattungsgemäßen Instrumentengriffen, elektrische Kontaktglieder vorgesehen zu sein brauchen. Dem Instrumentengriff 12 und damit auch dem Rohrschaftinstrument 10, können damit konstruktive einfachere Ausgestaltungen verliehen werden. Auch die Reinigung des Instrumentengriffes 12 wird durch Vermeidung von elektrischen Kontaktgliedern, insbesondere in dem vom Grundkörper 34 umgebenen Raum, erleichtert. Ebenso brauchen an dem in den Instrumentengriff 12 eingreifenden Abschnitt des Rohrschaftes 14 keine elektrischen Kontaktglieder vorhanden zu sein.

Die aus dem Rohr 24 ausgetretenen Abschnitte der Anschlussleitungen 68 und 70 weisen eine sie umgebende gemeinsame Umhüllung 78 auf. Die Umhüllung 78 ist längserstreckt und insbesondere geradlinig, zum Beispiel nach Art eines Kabelkanals. Die Umhüllung 78 fasst beide Anschlussleitungen 68 und 70 zu deren Schutz und zur vereinfachten Handhabung des Rohrschaftes 14 formschlüssig zusammen. Zum weiteren Schutz der Anschlussleitungen 68 und 70 ist die Umhüllung 78 biegesteif ausgebildet. Denkbar ist allerdings auch eine flexible, biegeweiche Ausgestaltung der Umhüllung.

Die Umhüllung 78 erstreckt sich ungefähr vom distalen Ende des Instrumentengriffes 12 bis zu dessen proximalen Ende, ungefähr bis zum Rand der Einführöffnung 60 des Verschlusselementes 44. Quer zur Griffachse 32 ist die Umhüllung 78 zwischen den Griffelementen 40 und 42 angeordnet. Die Anschlussleitungen 68 und 70 verlaufen infolgedessen parallel zur Griffachse 32, wobei die Umhüllung 78 proximalseitig das Verschlusselement 44 kontaktiert und im Übrigen in geringem Abstand zum Grundkörper 34 und zur Hülse 36 verläuft.

Die Umhüllung 78 ist derart zwischen den Griffelementen 40 und 42 positioniert, dass diese auch in ihrer an den Grundkörper 34 angenäherten Stellung zum Betätigen der Maulteile 22 die Umhüllung 78 nicht kontaktieren, so dass diese vom Benutzer als nicht störend bei der Handhabung des Instrumentengriffes 12 angesehen wird. Darüber hinaus ermöglicht dies eine kompakte Bauform des Rohrschaftinstrumentes 10 auch bei den außerhalb des Instrumentengriffes 12 geführten Anschlussleitungen 68 und 70.

Am proximalen Ende weist der Rohrschaft 14 ein an den Anschlussleitungen 68 und 70 angeordnetes Anschlusselement 80 auf. Das Anschlusselement 80 mit scheibenförmigem Körper 81 ist steckerförmig ausgestaltet mit zwei elektrischen Kontaktgliedern 82 und 83, die an der proximalen Stirnseite parallel zur Griffachse 32 aus dem Körper 81 austreten und im Übrigen in diesem gefasst sind. Beispielsweise sind die Kontaktglieder 82 und 83 in den Körper 81 eingegossen. Im Anschlusselement 80 steht je eines der Kontaktglieder 82 und 83 mit einer der Anschlussleitungen 68 bzw. 70 in elektrischer Verbindung. Um dies zu ermöglichen, sind die aus der Umhüllung 78 austretenden proximalen Enden der Anschlussleitungen 68 und 70 quer zur Griffachse 32 in den Körper 81 geführt, welcher zu diesem Zweck Bohrungen zur Aufnahme der Anschlussleitungen 68 und 70 aufweist.

Der Rohrschaft 14 kann über das Anschlusselement 80 mit einer Energieversorgung zum Bereitstellen elektrochirurgischer Energie verbunden werden, die über die Kontaktglieder 82 und 83 und die Anschlussleitungen 68 und 70 den Maulteilen 22 bereitgestellt werden kann.

Das, abgesehen von den Kontaktgliedern 82 und 83, einstückige Anschlusselement 80 bildet ein Verbindungselement 84 der bereits erwähnten Verbindungseinrichtung 50 aus. Das Anschlusselement 80 kann auf diese Weise durch Form- und Kraftschluss lösbar mit dem Verschlusselement 44 verbunden werden, um die aus dem Rohr 24 ausgetretenen proximalen Abschnitte der Anschlussleitungen 68 und 70 am Instrumentengriff 12 handhabungsfreundlich festzulegen. Das Verbinden des Anschlusselementes 80 mit und das Lösen von dem Verschlusselement 44 ist insbesondere auf benutzerfreundliche Weise werkzeuglos möglich.

Das Anschlusselement 80 umfasst zum Anliegen am Verschlusselement 44 ein Anlageglied 86, das planar ausgestaltet ist und an der distalen Stirnseite des Anschlusselementes 80 angeordnet ist. Das Anlageglied 86 definiert eine relativ zur Griffachse 32 geneigte Ebene, die parallel zur Lochachse 56 ausgerichtet ist und mit der vom Anlageglied 66 definierten Ebene zusammenfällt (Figur 7). Das Verschlusselement 44 und das Anschlusselement 80 können in miteinander verbundenem Zustand über die Anlageglieder 66 und 86 flanschartig aneinander anliegen und dadurch eine besonders definierte Relativorientierung zueinander einnehmen.

Vom Anlageglied 86 steht in distaler Richtung ein Vorsprung 88 des Anschlusselementes 80 ab, der als Rippe 90 ausgestaltet ist. Der Vorsprung 88 definiert eine Vorsprungachse 92, die relativ zur Griffachse 32 geneigt und parallel zur vom Anlageglied 86 definierten Ebene ausgerichtet ist.

Die Rippe 90 ist in Abmessung und Form an die Aufnahme 52 angepasst und weist einen im Sackloch 54 positionierbaren Kopfabschnitt 94 auf. Der Kopfabschnitt 94 ist von rundem Querschnitt und mit dem Anlageglied 86 über einen Halsabschnitt 96 des Vorsprunges 88 verbunden, der quer zur Griffachse 32 schmaler ist als der Kopfabschnitt 94.

Zum Verbinden des Anschlusselementes 80 mit dem Verschlusselement 44 kann der Vorsprung 88 in die Aufnahme 52 eingeführt werden (Figur 7), wobei der Kopfabschnitt 94 in das Sackloch 54 eingreift und der Halsabschnitt 96 in die Durchbrechung 64. Zwischen dem Verschlusselement 44 und dem Anschlusselement 80 ist auf diese Weise ein Form- und Kraftschluss zur zuverlässigen Verbindung gebildet, wobei die axiale Relativpositionierung außerdem durch die einander kontaktierenden Anlageglieder 66 und 86 sichergestellt ist. Der Vorsprung 88 und die Aufnahme 52 sichern das Anschlusselement 80 und das Verschlusselement 44 aneinander in allen drei Raumrichtungen axial längs der Griffachse 32 und quer dazu.

Durch die Neigung der zusammenfallenden Lochachse 56 und Vorsprungachse 92 relativ zur Griffachse 32 ist über den Formschluss des Vorsprungs 88 und der Aufnahme 52 hinaus ein Hinterschnitt zwischen dem Verschlusselement 44 und dem Anschlusselement 80 gebildet, die auf diese Weise zuverlässig gegen unbeabsichtigtes Lösen gesichert sind.

Die Anschlussleitungen 68 und 70 sind so bemessen, dass ihre aus dem Rohr 24 ausgetretenen Abschnitte frei von axialen Zug- und/oder Schubkräften sind, wenn das Verschlusselement 44 und das Anschlusselement 80 miteinander verbunden sind. Die Anschlussleitungen 68 und 70 sind dadurch insbesondere zugentlastet, so dass deren Beschädigung weitgehend vermieden werden kann.

Als vorteilhaft erweist es sich, dass sich die Umhüllung 78 distal nicht ganz bis zur Austrittsöffnung 72 erstreckt, so dass die Anschlussleitungen 68 und 70 im Bereich der Austrittsöffnung 72 gebogen oder geknickt werden können. Dies erleichtert einem Benutzer das Verbinden des Anschlusselementes 80 mit dem und das Lösen von dem Verschlusselement 44.

Das Verschlusselement 44 an seinem außerhalb des Grundkörpers 34 angeordneten Abschnitt und das Anschlusselement 80 sind bezüglich der Griffachse 32 zylindrisch ausgestaltet und hinsichtlich ihres Durchmessers und ihrer Außenkontur aneinander angepasst. Auf diese Weise fluchten das Anschlusselement 80 und das Verbindungselement 48 miteinander, so dass das Anschlusselement 80 als proximalseitige Verlängerung des Instrumentengriffes 12 angesehen werden kann. Dies verleiht dem Rohrschaftinstrument 10 zum einen ein gefälliges Aussehen und erleichtert zum anderen dessen Handhabung.

Die lösbare Verbindung des Verschlusselementes 44 und des Anschlusselementes 80 miteinander erweist sich als vorteilhaft, weil der Instrumentengriff 12 wiederverwendbar ist und mit einer Mehrzahl von Rohrschäften ein Wechselschaftsystem ausbilden kann. Nach dem Einsatz kann das Anschlusselement 80 des zu entsorgenden Rohrschaftes vom Instrumentengriff 12 gelöst werden und zusätzlich das Rohr 24 vom Instrumentengriff 12 getrennt werden. Ein weiterer Rohrschaft kann mit dem Instrumentengriff 12 verbunden werden, wobei dessen jeweiliges Anschlusselement 80 ebenfalls mit dem Verschlusselement 44 lösbar verbunden werden kann.

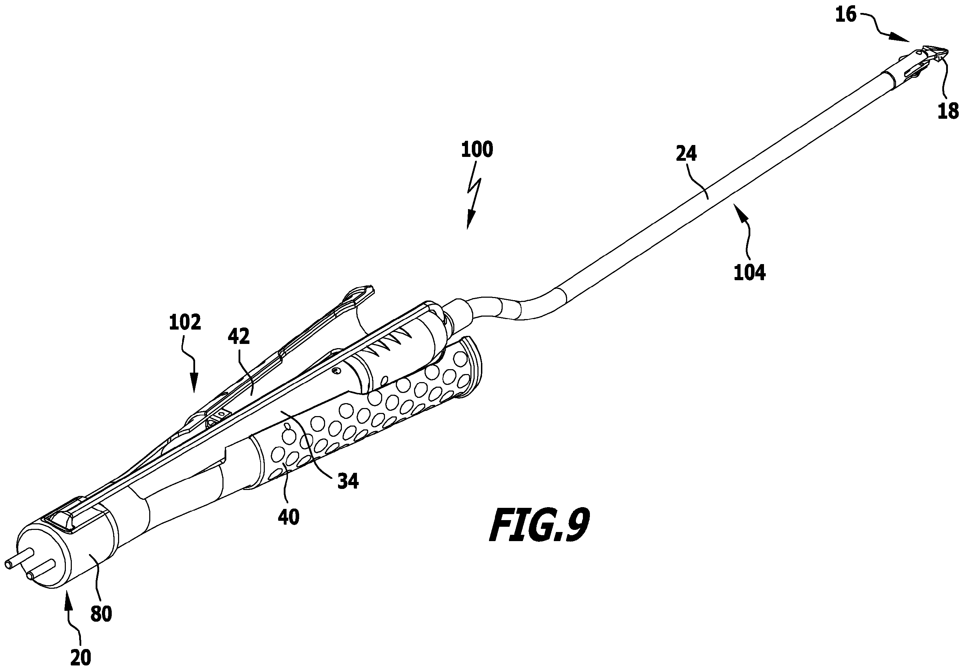

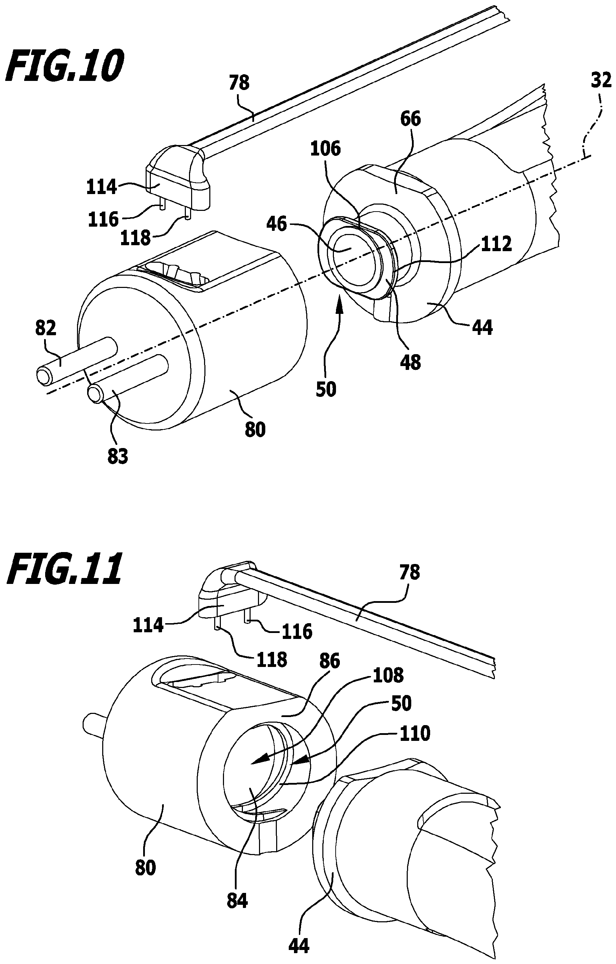

Die Figuren 9 bis 11 zeigen ganz bzw. teilweise eine weitere, insgesamt mit dem Bezugszeichen 100 belegte vorteilhafte Ausführungsform eines erfindungsgemäßen Rohrschaftinstrumentes. Das Rohrschaftinstrument 100 umfasst vorteilhafte Ausführungsformen eines erfindungsgemäßen Instrumentengriffes und eines erfindungsgemäßen Rohrschaftes, die mit den Bezugszeichen 102 bzw. 104 belegt sind.

Für gleiche oder gleichwirkende Merkmale und Bauteile der Rohrschaftinstrumente 10 und 100 werden identische Bezugszeichen benutzt. Die mit dem Rohrschaftinstrument 10 (sowie dem Instrumentengriff 12 und dem Rohrschaft 14) erzielbaren Vorteile können mit dem Rohrschaftinstrument 100 (und dementsprechend dem Instrumentengriff 102 und dem Rohrschaft 104) ebenfalls erzielt werden. Diesbezüglich kann auf voranstehende Erläuterungen verwiesen werden. Nachfolgend wird nur auf die wichtigsten Unterschiede eingegangen.

Der wichtigste Unterschied zwischen den Rohrschaftinstrumenten 10 und 100 besteht in der Ausgestaltung der Verbindungseinrichtung 50, über die das Anschlusselement 80 am Instrumentengriff 102 lösbar festlegbar ist. Das vom Verschlusselement 44 gebildete Verbindungselement 48 ist beim Instrumentengriff 102 ausgestaltet als Vorsprung 106 in Richtung des Anschlusselementes 80. Der Vorsprung 106 ist koaxial zur Griffachse 32 ausgerichtet und bildet einen Teil des Durchgangskanals 46.

Der Vorsprung 106 ist vorliegend ein Luer-Anschluss zum Anschließen einer Spülleitung, insbesondere ist der Vorsprung 106 zur Ausbildung einer Luer-Lock-Verbindung ausgestaltet. An den Instrumentengriff 102 kann von proximal eine Spülleitung angeschlossen werden, um den Instrumentengriff 102 nach dem Gebrauch und dem Lösen des Anschlusselementes 80 zu reinigen.

Beim Anschlusselement 80 ist das Verbindungselement 84 eine Aufnahme 108. Die Aufnahme 108 ist ein axial erstrecktes Sackloch, in die der Vorsprung 106 eingeführt werden kann. Zur Verbindung mit dem Vorsprung 106 ist die Aufnahme 108 komplementär zu diesem ausgestaltet, wodurch insbesondere eine Luer-Lock-Verbindung des Anschlusselementes 80 mit dem Verschlusselement 44 bereitgestellt werden kann. Hierzu kann das Anschlusselement 80 dem Verschlusselement 44 genähert und unter Drehung um die Griffachse 32 mit diesem durch Verschraubung verbunden werden. Ein Gewinde 110 an der Wandung der Aufnahme 108 kann mit einem quer zur Griffachse 32 ausgerichteten Fortsatz 112 des Vorsprungs 106 zusammenwirken.

Auch beim Rohrschaftinstrument 100 ist es möglich, das Anschlusselement 80 werkzeuglos mit dem Instrumentengriff 102 zu verbinden oder von diesem zu lösen.

Die Umhüllung 78 der in den Figuren 9 bis 11 nicht gezeigten Anschlussleitungen 68, 70 ist beim Rohrschaft 104 flexibel, beispielsweise ist sie eine flexible Schlauchleitung.

Proximalseitig an den Anschlussleitungen 68, 70 ist ein Steckglied 114 angeordnet mit Kontaktgliedern 116, 118. Durch Anschließen des Steckgliedes 114 an das Anschlusselement 80 können die Kontaktglieder 116, 118 in elektrische Verbindung mit den Kontaktgliedern 82 bzw. 83 gebracht werden und damit im Ergebnis die Anschlussleitungen 68, 70 elektrisch kontaktiert werden. Das Steckglied 114 kann vom Anschlusselement 80 gelöst werden.

Selbstverständlich ist denkbar, dass die Anschlussleitungen 68, 70 proximalseitig in das Anschlusselement 80 geführt sind, wie dies bei dem Rohrschaftinstrument 10 der Fall ist, ohne dass hierfür eine lösbare Verbindung über das Steckglied 114 vorgesehen ist.

## Patentansprüche

1. Elektrochirurgischer Rohrschaft (14; 104) für die Verwendung mit einem chirurgischen Instrumentengriff (12; 102) zur Ausbildung eines elektrochirurgischen Rohrschaftinstrumentes (10; 100), wobei der Rohrschaft (14; 104) ein Rohr (24) umfasst, ein distalseitig am Rohrschaft (14; 104) angeordnetes elektrochirurgisches Werkzeug (18), mindestens eine mit dem Werkzeug (18) in elektrischer Verbindung stehende und im Rohr (24) verlaufende elektrische Anschlussleitung (68, 70) sowie mindestens ein proximalseitig am Rohrschaft (14, 104) angeordnetes Kopplungselement (28) zum Zusammenwirken mit mindestens einem korrespondierenden Kopplungselement (37) des Instrumentengriffes (12; 102) zu dessen lösbarer Kopplung mit dem Rohrschaft (14; 104), wobei der Rohrschaft (14; 104) ein proximalseitig an der mindestens einen Anschlussleitung (68, 70) angeordnetes elektrisches Anschlusselement (80) für eine elektrische Energieversorgung umfasst, **dadurch gekennzeichnet, dass** das Rohr (24) eine distal des mindestens einen Kopplungselementes (28) angeordnete Austrittsöffnung (72) umfasst, durch die hindurch die mindestens eine Anschlussleitung (68, 70) aus dem Rohr (24) austritt.

2. Rohrschaft nach Anspruch 1, **dadurch gekennzeichnet, dass** die mindestens eine Anschlussleitung (68, 70) seitlich aus dem Rohr (24) austritt, quer zur einer vom Rohr (24) definierten Richtung.

3. Rohrschaft nach Anspruch 1 oder 2, **dadurch gekennzeichnet, dass** die Austrittöffnung (72) eine im Rohr (24) gebildete Durchbrechung (72) ist, durch die hindurch die mindestens eine Anschlussleitung (68, 70) aus dem Rohr (24) austritt.

4. Rohrschaft nach einem der voranstehenden Ansprüche, **dadurch gekennzeichnet, dass** der Rohrschaft (14; 104) eine das Rohr (24) aufnehmende Buchse (26) zum Einführen in eine korrespondierende Hülse (34, 36) des Instrumentengriffes (12; 102) umfasst, welche Buchse (26) proximalseitig zumindest ein Kopplungselement (28) umfasst, und dass in der Buchse (26) eine Durchbrechung (76) gebildet ist, durch die die mindestens eine Anschlussleitung (68, 70) hindurchgeführt ist.

5. Rohrschaft nach einem der voranstehenden Ansprüche, **dadurch gekennzeichnet, dass** der Rohrschaft (14; 104) eine die mindestens eine Anschlussleitung (68, 70) an deren aus dem Rohr (24) ausgetretenen Abschnitt umgebende biegesteife oder flexible Umhüllung (78) umfasst.

6. Rohrschaft nach einem der voranstehenden Ansprüche, **dadurch gekennzeichnet, dass** der Rohrschaft (14; 104) ein bipolarer elektrochirurgischer Rohrschaft (14; 104) ist und zwei mit dem Werkzeug (18) in elektrischer Verbindung stehende elektrische Anschlussleitungen (68, 70) umfasst, vorzugsweise dass ein gemeinsames Anschlusselement (80) vorgesehen ist, das proximalseitig an beiden Anschlussleitungen (68, 70) angeordnet ist.

7. Rohrschaft nach einem der voranstehenden Ansprüche, **dadurch gekennzeichnet, dass** der Rohrschaft (14; 104) mindestens ein im Anschlusselement (80) gefasstes, mit der mindestens einen Anschlussleitung (68, 70) elektrisch verbundenes Kontaktglied (82, 83) aufweist.

8. Rohrschaft nach einem der voranstehenden Ansprüche, **dadurch gekennzeichnet, dass** der Rohrschaft (14; 104) mindestens ein Verbindungselement (84) umfasst zum lösbaren Festlegen der mindestens einen Anschlussleitung (68, 70) am Instrumentengriff (12; 102).

9. Rohrschaft nach Anspruch 8, **dadurch gekennzeichnet, dass** das Anschlusselement (80) ein Verbindungselement (84) umfasst oder ausbildet, vorzugsweise dass das Verbindungselement (84) einstückig mit dem Anschlusselement (80) gebildet ist.

10. Rohrschaft nach Anspruch 8 oder 9, **dadurch gekennzeichnet, dass** das mindestens eine Verbindungselement (84) ein Anlageglied (86) für den Instrumentengriff (12) umfasst oder ausbildet, insbesondere an einer distalen Stirnseite.

11. Chirurgischer Instrumentengriff (12; 102) zur Verwendung mit einem elektrochirurgischen Rohrschaft (14; 104) nach einem der voranstehenden Ansprüche zur Ausbildung eines elektrochirurgischen Rohrschaftinstrumentes (10; 100), umfassend mindestens ein Kopplungselement (37) zum Zusammenwirken mit mindestens einem korrespondierenden Kopplungselement (28) des Rohrschaftes (14; 104) zu dessen lösbarer Kopplung mit dem Instrumentengriff (12; 102), sowie mindestens ein Verbindungselement (48) zum lösbaren Festlegen der mindestens einen Anschlussleitung (68, 70) des Rohrschaftes (14; 104) am Instrumentengriff (12; 102), wobei das mindestens eine Verbindungselement (48) proximalseitig am Instrumentengriff (12; 102) angeordnet ist, insbesondere an einer proximalen Stirnseite des Instrumentengriffes (12; 102), **dadurch gekennzeichnet, dass** der Instrumentengriff (12; 120) frei von innenliegenden elektrischen Kontaktgliedern zur elektrischen Kontaktierung des elektrochirurgischen Rohrschaftes ist.

12. Elektrochirurgisches Rohrschaftinstrument, umfassend einen chirurgischen Instrumentengriff (12; 102) mit mindestens einem Kopplungselement (37) und mindestens einem Verbindungselement (48) sowie einen elektrochirurgischen Rohrschaft (14; 104) nach einem der Ansprüche 1 bis 10, der lösbar mit dem Instrumentengriff (12; 102) verbindbar ist, wobei das mindestens eine Kopplungselement (37) des Instrumentengriffes (12; 102) mit dem mindestens einem korrespondierenden Kopplungselement (28) des Rohrschaftes (14; 104) zu dessen lösbarer Kopplung mit dem Instrumentengriff (12; 102) zusammenwirkt, und wobei der Rohrschaft (14; 104) mindestens ein Verbindungselement (84) umfasst zum lösbaren Festlegen der mindestens einen Anschlussleitung (68, 70) am Instrumentengriff (12; 102), das mit dem mindestens einen korrespondierend dazu ausgebildeten Verbindungselement (48) des Instrumentengriffes (12; 102) zusammenwirkt.

13. Rohrschaftinstrument nach Anspruch 12, **dadurch gekennzeichnet, dass** die mindestens eine Anschlussleitung (68, 70) so bemessen ist, dass sie mit ihrem aus dem Rohr (24) ausgetretenen Abschnitt frei oder im Wesentlichen frei von axialen Zug- und/oder Schubkräften ist.

14. Rohrschaftinstrument nach Anspruch 12 oder 13, **dadurch gekennzeichnet, dass** der aus dem Rohr (24) ausgetretene Abschnitt der mindestens einen Anschlussleitung (68, 70) längs des Instrumentengriffes (12; 102) achsparallel von distal nach proximal verläuft.

15. Rohrschaftinstrument nach einem der Ansprüche 12 bis 14, **dadurch gekennzeichnet, dass** der aus dem Rohr (24) ausgetretene Abschnitt der mindestens einen Anschlussleitung (68, 70) quer zur Axialrichtung seitlich neben einem betätigbaren Griffelement (40, 42) des Instrumentengriffes (12) zum Einwirken auf das Werkzeug (18) verläuft.

## Claims

1. Electrosurgical tubular shaft (14; 104) for use with a surgical instrument grip (12; 102) for formation of an electrosurgical tubular shaft instrument (10; 100), the tubular shaft (14; 104) comprising a tube (24), an electrosurgical tool (18) arranged at the distal end on the tubular shaft (14; 104), at least one electric connection line (68, 70) electrically connected to the tool (18) and extending in the tube (24), and at least one coupling element (28) arranged at the proximal end on the tubular shaft (14; 104) for interacting with at least one corresponding coupling element (37) of the instrument grip (12; 102) for releasably coupling the instrument grip (12; 102) to the tubular shaft (14; 104), the tubular shaft (14; 104) comprising an electric connection element (80) for an electric energy supply, the electric connection element (80) being arranged at the proximal end on the at least one connection line (68, 70), **characterized in that** the tube (24) comprises an outlet opening (72) which is arranged distally of the at least one coupling element (28) and through which the at least one connection line (68, 70) issues from the tube (24).

2. Tubular shaft in accordance with claim 1, **characterized in that** the at least one connection line (68, 70) issues at the side from the tube (24), transversely to a direction defined by the tube (24).

3. Tubular shaft in accordance with claim 1 or 2, **characterized in that** the outlet opening (72) is a through-opening (72) which is formed in the tube (24) and through which the at least one connection line (68, 70) issues from the tube (24).

4. Tubular shaft in accordance with any one of the preceding claims, **characterized in that** the tubular shaft (14; 104) comprises a bushing (26), receiving the tube (24), for insertion into a corresponding sleeve (34, 36) of the instrument grip (12; 102), the bushing (26) comprising at the proximal end at least one coupling element (28), and **in that** there is formed in the bushing (26) a through-opening (76) through which the at least one connection line (68, 70) is led.

5. Tubular shaft in accordance with any one of the preceding claims, **characterized in that** the tubular shaft (14; 104) comprises a sheath (78) which is resistant to bending or flexible and surrounding the at least one connection line (68, 70) at its section which has issued from the tube (24).

6. Tubular shaft in accordance with any one of the preceding claims, **characterized in that** the tubular shaft (14; 104) is a bipolar electrosurgical tubular shaft (14; 104) and comprises two electric connection lines (68, 70) electrically connected to the tool (18), preferably **in that** a common connection element (80) is provided, which is arranged at the proximal end on both connection lines (68, 70).

7. Tubular shaft in accordance with any one of the preceding claims, **characterized in that** the tubular shaft (14; 104) comprises at least one contact member (82, 83) which is accommodated in the connection element (80) and is electrically connected to the at least one connection line (68, 70).

8. Tubular shaft in accordance with any one of the preceding claims, **characterized in that** the tubular shaft (14; 104) comprises at least one connecting element (84) for releasably fixing the at least one connection line (68, 70) to the instrument grip (12; 102).

9. Tubular shaft in accordance with claim 8, **characterized in that** the connection element (80) comprises or forms a connecting element (84), preferably **in that** the connecting element (84) is formed in one piece with the connection element (80).

10. Tubular shaft in accordance with claim 8 or 9, **characterized in that** the at least one connecting element (84) comprises or forms an abutment member (86) for the instrument grip (12), in particular, on a distal end face.

11. Surgical instrument grip (12; 102) for use with an electrosurgical tubular shaft (14; 104) in accordance with any one of the preceding claims for formation of an electrosurgical tubular shaft instrument (10; 100), comprising at least one coupling element (37) for interacting with at least one corresponding coupling element (28) of the tubular shaft (14; 104) for releasably coupling the tubular shaft (14; 104) to the instrument grip (12; 102), and at least one connecting element (48) for releasably fixing the at least one connection line (68, 70) of the tubular shaft (14; 104) to the instrument grip (12; 102), the at least one connecting element (48) being arranged at the proximal end on the instrument grip (12; 102), in particular, on a proximal end face of the instrument grip (12; 102), **characterized in that** the instrument grip (12; 102) is free of inside electric contact members for electric contacting of the electrosurgical tubular shaft.

12. Electrosurgical tubular shaft instrument, comprising a surgical instrument grip (12; 102) with at least one coupling element (37) and at least one connecting element (48) and an electrosurgical tubular shaft (14; 104) in accordance with any one of claims 1 to 10, which is releasably connectable to the instrument grip (12; 102), the at least one coupling element (37) of the instrument grip (12; 102) interacting with the at least one corresponding coupling element (28) of the tubular shaft (14; 104) for releasably coupling the tubular shaft (14; 104) to the instrument grip (12; 102), and the tubular shaft (14; 104) comprising at least one connecting element (84) for releasably fixing the at least one connection line (68, 70) to the instrument grip (12; 102), which interacts with the at least one correspondingly constructed connecting element (48) of the instrument grip (12; 102).

13. Tubular shaft instrument in accordance with claim 12, **characterized in that** the at least one connection line (68, 70) is of such dimensions that with its section which has issued from the tube (24) it is free or substantially free of axial pulling and/or pushing forces.

14. Tubular shaft instrument in accordance with claim 12 or 13, **characterized in that** the section of the at least one connection line (68, 70) which has issued from the tube (24) extends axially parallel from distal to proximal along the instrument grip (12; 102).

15. Tubular shaft instrument in accordance with any one of claims 12 to 14, **characterized in that** the section of the at least one connection line (68,70) which has issued from the tube (24) extends, transversely to the axial direction, alongside an actuatable grip element (40, 42) of the instrument grip (12) for acting on the tool (18).

## Revendications

1. Tige tubulaire électrochirurgicale (14 ; 104) destinée à être utilisée avec une poignée d'instrument chirurgicale (12 ; 102) pour la formation d'un instrument à tige tubulaire électrochirurgical (10 ; 100), où la tige tubulaire (14 ; 104) comprend un tube (24), un outil électrochirurgical (18) disposé du côté distal sur la tige tubulaire (14 ; 104), au moins une ligne de raccordement électrique (68, 70) en liaison électrique avec l'outil (18) et s'étendant dans le tube (24) ainsi qu'au moins un élément de couplage (28) disposé du côté proximal sur la tige tubulaire (14 ; 104) destiné à coopérer avec au moins un élément de couplage (37) correspondant de la poignée d'instrument (12 ; 102) pour son couplage amovible avec la tige tubulaire (14 ; 104), où la tige tubulaire (14 ; 104) comprend un élément de raccordement électrique (80) disposé du côté proximal sur la au moins une ligne de raccordement (68, 70) pour une alimentation en énergie électrique, **caractérisée en ce que** le tube (24) comprend une ouverture de sortie (72) disposée de manière distale par rapport au au moins un élément de couplage (28), à travers laquelle la au moins une ligne de raccordement (68, 70) sort du tube (24).

2. Tige tubulaire selon la revendication 1, **caractérisée en ce que** la au moins une ligne de raccordement (68, 70) sort latéralement du tube (24), transversalement par rapport à une direction définie par le tube (24).

3. Tige tubulaire selon la revendication 1 ou 2, **caractérisée en ce que** l'ouverture de sortie (72) est un perçage (72) formé dans le tube (24) à travers lequel la au moins une ligne de raccordement (68, 70) sort du tube (24).

4. Tige tubulaire selon l'une des revendications précédentes, **caractérisée en ce que** la tige tubulaire (14 ; 104) comprend une douille (26) recevant le tube (24) pour l'introduction dans une gaine (34, 36) correspondante de la poignée d'instrument (12 ; 102), laquelle douille (26) comprend du côté proximal au moins un élément de couplage (28), et **en ce que** dans la douille (26) est formée un perçage (76) à travers lequel est amenée à passer la au moins une ligne de raccordement (68, 70).

5. Tige tubulaire selon l'une des revendications précédentes, **caractérisée en ce que** la tige tubulaire (14 ; 104) comprend une enveloppe (78) rigide en flexion ou flexible qui entoure la au moins une ligne de raccordement (68, 70) au niveau de son segment sortant du tube (24).

6. Tige tubulaire selon l'une des revendications précédentes, **caractérisée en ce que** la tige tubulaire (14 ; 104) est une tige tubulaire électrochirurgicale bipolaire (14 ; 104) et comprend deux lignes de raccordement électriques (68, 70) en liaison électrique avec l'outil (18), de préférence **en ce qu'**il est prévu un élément de raccordement (80) commun, qui est disposé du côté proximal sur les deux lignes de raccordement (68, 70).

7. Tige tubulaire selon l'une des revendications précédentes, **caractérisée en ce que** la tige tubulaire (14 ; 104) présente au moins un organe de contact (82, 83) monté dans l'élément de raccordement (80), relié électriquement à la au moins une ligne de raccordement (68, 70).

8. Tige tubulaire selon l'une des revendications précédentes, **caractérisée en ce que** la tige tubulaire (14 ; 104) comprend au moins un élément de liaison (84) pour la fixation amovible de la au moins une ligne de raccordement (68, 70) à la poignée d'instrument (12 ; 102).

9. Tige tubulaire selon la revendication 8, **caractérisée en ce que** l'élément de raccordement (80) comprend ou forme un élément de liaison (84), de préférence **en ce que** l'élément de liaison (84) est formé d'une pièce avec l'élément de raccordement (80).

10. Tige tubulaire selon la revendication 8 ou 9, **caractérisée en ce que** le au moins un élément de liaison (84) comprend ou forme un organe d'appui (86) pour la poignée d'instrument (12), en particulier au niveau d'un côté frontal distal.

11. Poignée d'instrument chirurgicale (12 ; 102) destinée à être utilisée avec une tige tubulaire électrochirurgicale (14 ; 104) selon l'une des revendications précédentes pour la formation d'un instrument à tige tubulaire électrochirurgical (10 ; 100), comprenant au moins un élément de couplage (37) destiné à coopérer avec au moins un élément de couplage (28) correspondant de la tige tubulaire (14 ; 104) pour son couplage amovible avec la poignée d'instrument (12 ; 102), ainsi qu'au moins un élément de liaison (48) pour la fixation amovible de la au moins une ligne de raccordement (68, 70) de la tige tubulaire (14 ; 104) sur la poignée d'instrument (12 ; 102), où le au moins un élément de liaison (48) est disposé du côté proximal sur la poignée d'instrument (12 ; 102), en particulier au niveau d'un côté frontal proximal de la poignée d'instrument (12 ; 102), **caractérisée en ce que** la poignée d'instrument (12 ; 120) est dépourvue d'organes de contact électriques internes pour la mise en contact électrique de la tige tubulaire électrochirurgicale.

12. Instrument à tige tubulaire électrochirurgical comprenant une poignée d'instrument chirurgicale (12 ; 102) avec au moins un élément de couplage (37) et au moins un élément de liaison (48) ainsi qu'une tige tubulaire électrochirurgicale (14 ; 104) selon l'une des revendications 1 à 10, qui peut être reliée de manière amovible à la poignée d'instrument (12 ; 102), où le au moins un élément de couplage (37) de la poignée d'instrument (12 ; 102) coopère avec le au moins un élément de couplage (28) correspondant de la tige tubulaire (14 ; 104) pour son couplage amovible avec la poignée d'instrument (12 ; 102), et où la tige tubulaire (14 ; 104) comprend au moins un élément de liaison (84) pour la fixation amovible de la au moins une ligne de raccordement (68, 70) sur la poignée d'instrument (12 ; 102), qui coopère avec le au moins un élément de liaison (48) de la poignée d'instrument (12 ; 102) formé de manière correspondante à celui-ci.

13. Instrument à tige tubulaire selon la revendication 12, **caractérisé en ce que** la au moins une ligne de raccordement (68, 70) est dimensionnée de telle sorte qu'elle est dépourvue ou sensiblement dépourvue de forces de traction et/ou de poussée axiales au niveau de son segment sortant du tube (24).

14. Instrument à tige tubulaire selon la revendication 12 ou 13, **caractérisé en ce que** le segment sortant du tube (24) de la au moins une ligne de raccordement (68, 70) s'étend de distal à proximal le long de la poignée d'instrument (12 ; 102) parallèlement à l'axe.

15. Instrument à tige tubulaire selon l'une des revendications 12 à 14, **caractérisé en ce que** le segment sortant du tube (24) de la au moins une ligne de raccordement (68, 70) s'étend, transversalement par rapport à la direction axiale, latéralement à côté d'un élément de poignée (40, 42) de la poignée d'instrument (12) qui peut être actionné pour agir sur l'outil (18).
